# EUROPEAN PATENT SPECIFICATION

(11) **EP 0 821 739 B1**
(45) Date of publication and mention of the grant of the patent: **18.06.2003**
(21) Application number: 96912839.6
(22) Date of filing: 17.04.1996
(51) Int. Cl.: C12N 15/861

(54) **AN ADENOVIRUS HELPER-VIRUS SYSTEM**
ADENOVIRUS HELFERVIRUS SYSTEM
SYSTEME ADENOVIRALE A VIRUS AUXILIAIRES

(30) Priority: 17.04.1995 US 423573
(43) Date of publication of application: 04.02.1998
(73) Proprietor: BOARD OF REGENTS THE UNIVERSITY OF TEXAS SYSTEM, Austin, Texas 78701 (US)
(72) Inventor: ZHANG, Wei-Wei, Libertyville, IL 60048 (US); ALEMANY, Ramon, Grayslake, IL 60030 (US)
(74) Representative: Grünecker, Kinkeldey, Stockmair & Schwanhäusser Anwaltssozietät
(86) International application number: US9605310
(87) International publication number: WO96033280

(56) References cited:
- WO-A-94/28152
- WO-A-95/02697
- WO-A-95/29993
- WO-A-95/34671

## Description

### BACKGROUND OF THE INVENTION

### 1. Field of the Invention

The present invention relates generally to the field of viral vectors, helper viruses and the use of such viral vectors and helper viruses to express foreign DNA in mammalian cells. The invention also relates to the field of gene therapy and, particularly, gene therapy involving viral vectors to import genetic material to particular tissues *in vivo*. More particularly, the invention relates (i) to the genetic engineering of adenovirus to displace a large amount of the adenoviral genome with heterologous DNA, (ii) to the propagation of adenoviral vectors using a helper cell line and one or more helper viruses which complement replicative defects in the vectors and (iii) the infection of mammalian cells with such vectors to express a heterologous, non-adenoviral product that is therapeutic for some diseases.

### 2. Description of the Related Art

Gene therapy is an area that offers an attractive alternative for the treatment of many diseases and disorders. In particular, the ability of viruses to enter a cell and express its genetic material in the host cell raises the possibility of replacing lost or defective gene function *in vivo*. However, for gene therapy to succeed, there is a need for new vectors with the properties of high therapeutic index, large capacity, targeted gene delivery and tissue-specific gene expression. Currently available gene transfer vectors are not able to meet the requirement of high therapeucic index (Mulligan, 1993), however, because of the vector-borne cyto- or geno-toxicities that are associated therewith.

Multiple and targeted gene transfer is particularly relevant to gene therapy for cancer (Friedmann, 1992). Throughout the last decade, studies of oncogenes and tumor suppressor genes have revealed more and more evidence that cancer is a disease developed through a process of multiple cytogenetic disorders (Chiao *et al*., 1990; Levine, 1990; Weinberg, 1991; Sugimura *et al*., 1992). Based on this concept of carcinogenesis, new strategies have developed rapidly as alternatives to conventional cancer therapy (Renan, 1990; Lotze *et al*., 1992; Pardoll, 1992). One of these is gene therapy (Friedmann, 1989), in which tumor suppressor genes, antisense oncogenes, and other related genes are used as therapeutic genes. It is believed that to achieve a maximal therapeutic effect, targeted delivery of a combination of these therapeutic genes by a single higher-capacity vector into cancer cells will be essential. Unfortunately, vector currently available vector technology is limited in this regard.

Adeno-associated virus (AAV) has recently been developed as a gene transfer system. Wild-type AAV has high infectivity and specificity in integrating into the host cell genome (Hermonat and Muzyczka, 1984; Lebkowski *et al*., 1988). However, experimental data has shown that recombinant AAV tend to have low titers and lose their specificity of integration (Samulski *et al.*, 1989). Also, the maximum gene-carrying capacity for AAV is under 5 kB (Walsh *et al*., 1992).

Adenoviruses (Ad) have been widely studied and well-characterized as a model system for eukaryotic gene expression. Ad are easy to grow and manipulate, and they exhibit broad host range *in vitro* and *in vivo.* This group of viruses can be obtained in high titers, *e.g*., 10⁹-10¹¹ plaque-forming unit (PFU)/ml, and they are highly infective. Adenoviruses are not, however, associated with any significant pathologies.

The life cycle of Ad does not require integration into the host cell genome. The foreign genes delivered by Ad vectors are expressed episomally and, therefore, have low genotoxicity to host cells. Ad appear to be linked only to relatively mild diseases since there is no known association of human malignancies with Ad infection. Moreover, no side effects have been reported in studies of vaccination with wild-type Ad (Couch *et al*., 1963; Top *et al.,* 1971), demonstrating their safety and therapeutic potential as *in vivo* gene transfer vectors.

Ad vectors have been successfully used in eukaryotic gene expression (Levrero *et al.,* 1991; Gomez-Foix *et al.,* 1992) and vaccine development (Grunhaus and Horwitz, 1992; Graham and Prevec, 1992). Recently, animal studies demonstrated that recombinant Ad could be used for gene therapy (Stratford-Perricaudet and Perricaudet, 1991; Stratford-Perricaudet *et al*., 1990; Rich *et al*., 1993). Successful experiments in administering recombinant Ad to different tissues include trachea instillation (Rosenfeld *et al*., 1991; Rosenfeld *et al*., 1992), muscle injection (Ragot *et al*., 1993), peripheral intravenous injection (Herz and Gerard, 1993), and stereotactic inoculation into the brain (Le Gal La Salle *et al*., 1993).

Generation and propagation of the current Ad vectors depend on a unique helper cell line, designated 293, which was transformed from human embryonic kidney cells by Adenovirus serotype 5 (Ad5) DNA fragments and constitutively expresses E1 proteins (Graham *et al*., 1977). Since the E3 region is dispensable from the Ad genome (Jones and Shenk, 1978), the current Ad vectors, with the help of 293 cells, carry foreign DNA in either the E1, the E3 or both regions (Graham and Prevec, 1991; Bett *et al.,* 1994).

In nature, Ad can package approximately 105% of the wild-type genome (Ghosh-Choudhury, *et al*., 1987), providing capacity for about 2 extra kB of DNA. Combined with the approximately 5.5 kB of DNA that is replaceable in the E1 and E3 regions, the maximum capacity of the known Ad vectors is under 7.5 kB, or about 15% of the. total length of the vector. In addition to preventing further insertion of heterologous DNA, the remaining 80% of the Ad genome is the source of vector-borne cytotoxicity. Also, the replication deficiency in E1-defective virus is incomplete and leakage of viral gene expression has been observed with the currently available Ad vectors at high multiplicities of infection (Mulligan, 1993).

WO 94/28152 discloses adenoviral vectors containing adenoviral inverted terminal repeats and an adenoviral packaging signal, but having partial or complete deletions in the E1A, E1B, E2, E3 and/or E4 coding regions, and complementation cell lines for replication and packaging of the defective adenoviral vectors. The adenoviral vectors disclosed in WO 95/02697 contain adenoviral inverted terminal repeats and an adenoviral packaging signal, but the E1 gene and at least one of the genes E2, E4 and L1-L5 are not functional. These coding regions can be replaced by up to 30 kB heterologous DNA that can be expressed under the control of a eukaryotic or viral promoter. Adenoviral vectors that aredeficient in two or more adenoviral regions are disclosed in WO 95/34671.

Another problem with the currently available adenovirus vectors is the potential for generation of wild-type virus by recombination. This may occur because the left end of the current Ad vectors contains a sequence of about 1.5 kB (9.8-14 map units) overlapping with the E1 fragment in 293 cells (Graham, *et al*., 1977). Homologous recombinations that generate wild-type virus were detectable when E1 substitution vectors were extensively amplified in 293 cells (personal communication, Dr. Richard Gregory, CANJI, Inc., San Diego, CA).

WO 95/29993 discloses gene delivery vectors derived from an adenovirus. The vectors contain adenoviral inverted repeats, an adenoviral packaging signal and heterologous DNA under the control of a viral promoter. Packaging cell lines and a virus system for replication and packaging of the defective adenoviral vectors are also disclosed.

However, there still exists an immediate need for an adenoviral vector system that will have a high therapeutic index, a large carrying capacity for heterologous DNA and the capacity for targeted gene delivery and tissue specific expression. Such a vector system will have utility in a wide variety of *in vivo* and *in vitro* applications such a gene therapy protocols, the production of useful protein products in mammalian cell culture, as gene transfer markers or for the diagnosis of genetic deficiencies in particular cell lines.

### SUMMARY OF THE INVENTION

The present invention seeks to overcome these and other drawbacks inherent in the prior art by using an adenoviral "helper virus" system wherein regions of adenovirus are eliminated from vectors and provided, in *trans,* by helper viruses. Vectors having multigene deletions have an extremely large capacity for carrying recombinant genetic material.

Helper viruses which, in conjunction with helper cell lines, are capable of providing all essential adenoviral functions in trans, have been developed.

By supporting displacement of more than 95% of the adenoviral genome, cell lines and helper viruses make possible the construction and propagation of a vector lacking all but the adenoviral inverted terminal repeats (ITRs) and packaging signals. This, in turn, permits incorporation of up to 35 kB of heterologous DNA into the vector and reduces cytotoxicity from the expression of adenoviral gene products.

The present invention provides a virion particle containing a packaged adenovirus vector construct, wherein said vector construct comprises an adenoviral inverted terminal repeat and an adenoviral packaging signal but lacks at least a portion of the coding regions for (i)the adenoviral products E1A, E1B and E3; and (ii) at least one of the adenoviral products selected from the group consisting of E2, E4, L1, L2, L3, L4 and L5, said virion particle being modified to incorporate a cell targeting agent that is encoded by a gene inserted into the vector construct. In a preferred embodiment, the vector lacks all adenoviral coding regions. Such vectors can carry about 10, 15, 20, 30 or 35 kB of foreign DNA.

In another embodiment, there is provided a virion particle containing a packaged adenovirus vector construct, wherein said vector construct comprises an adenoviral inverted terminal repeat and an adenoviral packaging signal and a non-functional, non-immunogenic form of (i) the adenoviral products E1A, E1B and E3; and (ii) at least one of the adenoviral products selected from the group consisting of E2, E4, L1, L2, L3, L4 and L5, said virion particle being modified to incorporate a cell targeting agent that is encoded by a gene inserted into the vector construct.

In a further embodiment of the invention, the gene encoding the cell targeting agent is inserted into a viral capsid gene. The cell targeting agent can be an antibody, a cell receptor recognition peptide, a binding rite peptide or a ligand peptide. Preferably, the cell targeting agent gene encodes an Fc binding region, a ligand binding site or a cell specific epitope.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein:

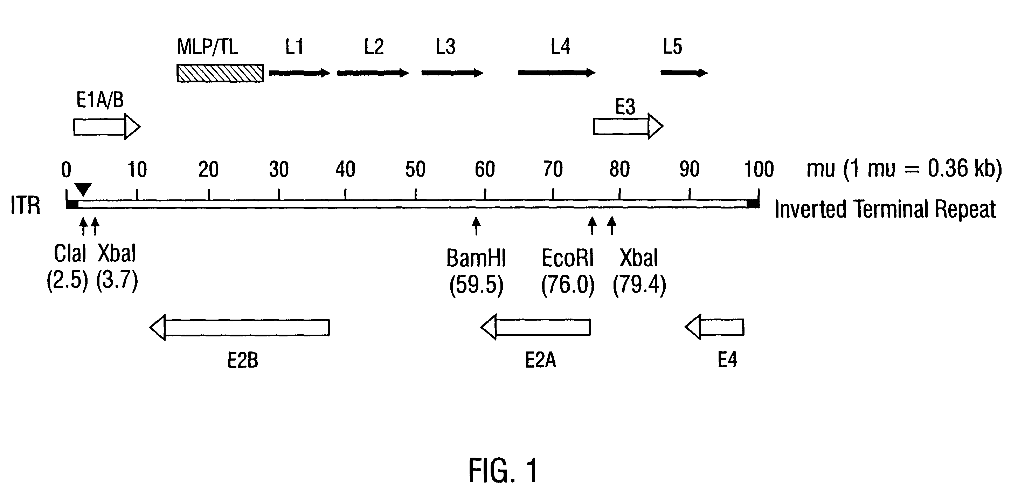

**FIG. 1.** This figure dspicts the structure of the Ad5 genome. The genome is divided into 100 map units (mu). The open arrows represent early (E) transcription and the solid arrows represent late (L) transcription. The direction of transcription is indicated by arrows. Gaps in arrows indicate intervening sequences. The hatched box represents location of major late promoter and tripartite leader sequences (MLP/TL). The numbers in parenthesis indicate the map units.

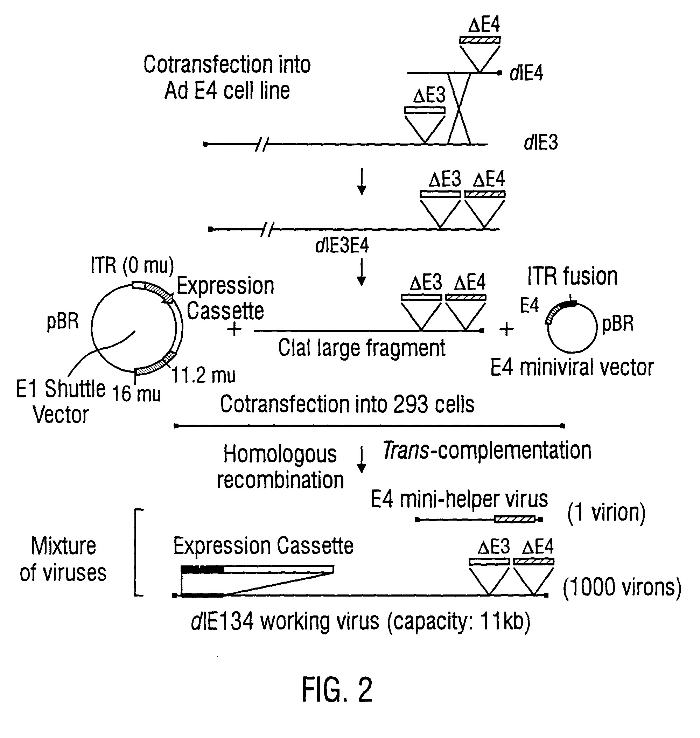

**FIG. 2.** This figure depicts the general scheme of for creating a defective adenoviral vector containing a heterologous expression cassette. Briefly, a large fragment from the left of an E3⁻ adenovirus is cotransfected with the right end of an E4⁻ adenovirus in an E4-expressing cell line. The resulting virus *dl*E3E4 is cleaved with *Cla*I to generate a fragment that contains the right end of the genome with the E3 and E4 deletions. An E1 shuttle vector contains the left ITR and packaging signal (0-1.25 map units), a multiple cloning site for insertion of an expression cassette and map units 11.2-16 to permit homologous recombination. An E4 minivirus contains the left ITR, the packaging signal and the entire E4 region with the right ITR (90-100 map units). To prevent homologous recombination between the E4 minivirus and *dl*E3E4, the constructs are designed to have no overlapping sequences at the beginning of the E4 region (90 map units). The E1 shuttle vector, the *Cla*I fragment of *dl*E3E4 and the E4 minivirus are transfected into E1-expressing cells, homologous recombination occurs between the *Cla*I fragment and the E1 shuttle vector and the E4 minivirus provides E4 product to complement the E4 defect in the recombinant.

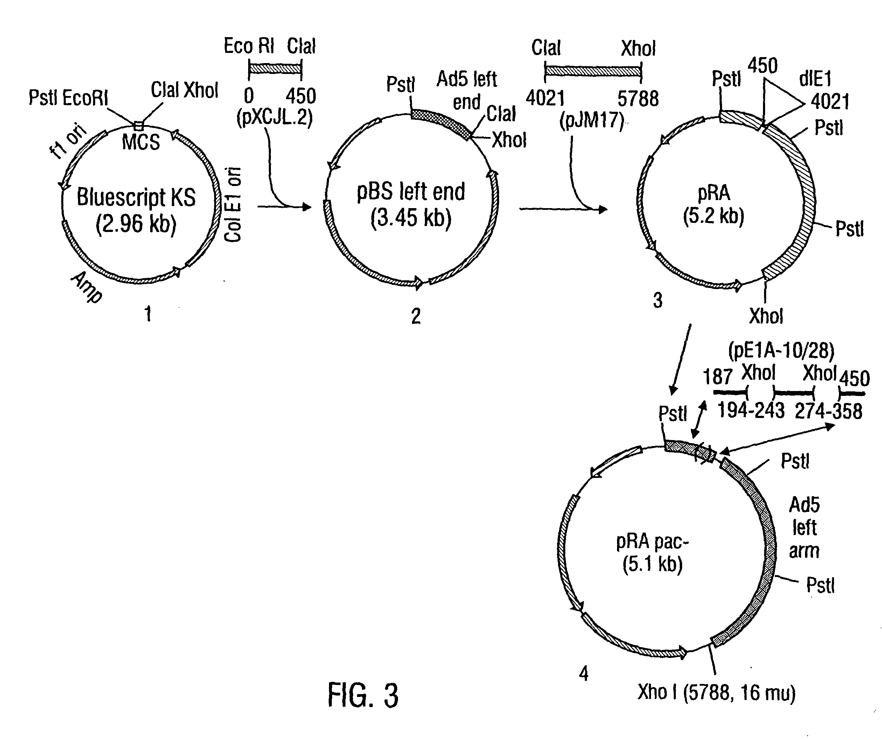

**FIG. 3.** This figure depicts the generation of pRApac⁻. The left end of Ad5 (0-450 bp) was cloned into Bluescript. The Ad5 sequence from 4021 bp (with a *Cla*I linker addition) to 5788 (*Xho*I site) was cloned downstream of this fragment, generating an E1 deletion (*dl*E1 450-4021 bp; plasmid 3). This deletion is 700 base pairs longer than previously reported E1 deletions, reducing the possibility of recombination with the E1 region in 293 cells; the complete sequence of protein IX is removed. As protein IX mutants have a restriction in the size of viral DNA they can package, only helper virus with an E1 deletion will be packaged. The Ad5 packaging signal present in this plasmid (194-358 bp of the Ad5 left end) was substituted by a mutated signal obtained from plasmid pE1A-10/28 The resultant plasmid can be used to produce a helper virus doubly-defective in E1 and the packaging signal.

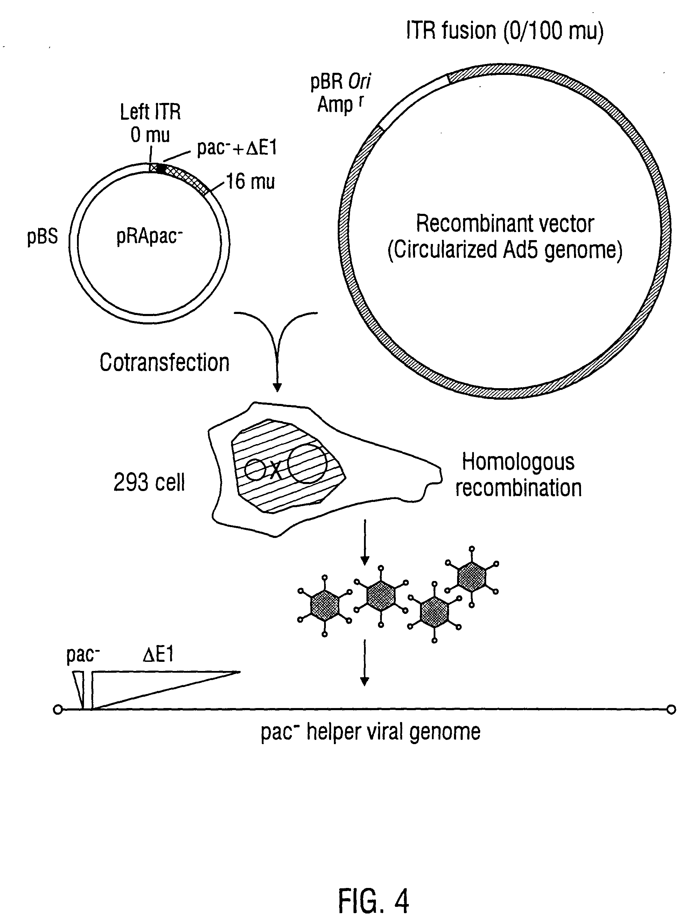

**FIG. 4.** To generate the packaging attenuated helper virus, the pac⁻ pRA shuttle vector is contransfected in to E1-expressing cells with an Ad recombinant vector, such as pJM17. The pac⁻ shuttle vector contains a partial deletion of the packaging signal and complete deletion of the E1 region. The recombinant vector contains the entire Ad5 genome circularized through fusion of ITR with an insertion of pBR322 plasmid in the E1 region. Homologous recombination occurs between pRApac⁻ and the circularized Ad5 genome to generate the pac⁻ helper virus. This E1⁻, pac⁻ helper virus can be propagated in E1-expressing cells to make stocks of the virus.

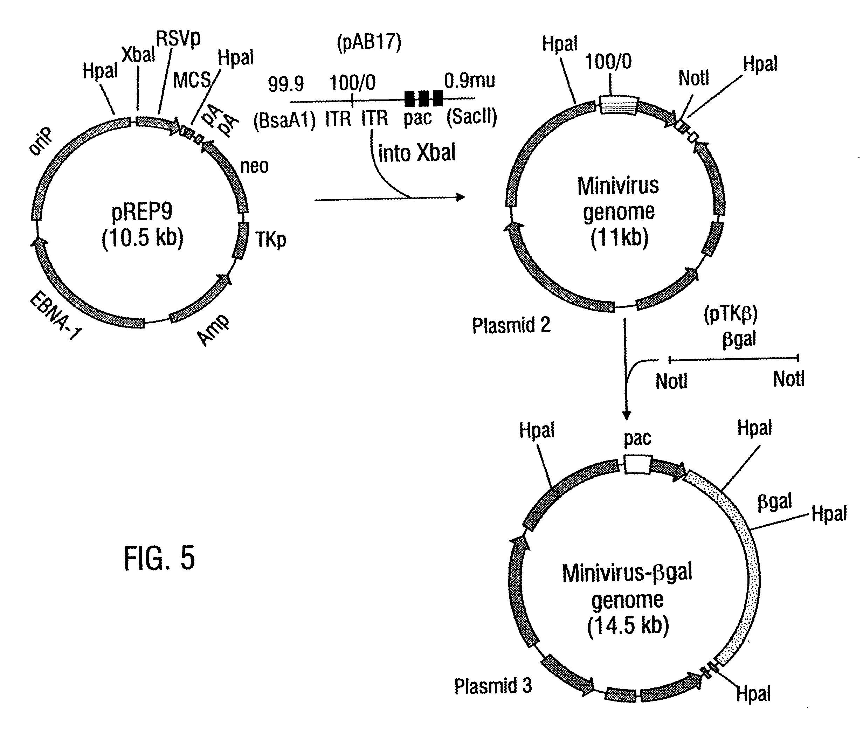

**FIG. 5.** This figure depicts the construction of the adenoviral vector or "minivirus." A fragment of 0.5 kB (*Bsa*AI-*Sac*II) containing the fused ITR of Ad5 was cloned into the *Xba*I site of pREP9 (Invitrogen, San Diego, CA). Plasmid 2 (minivirus genome) is the miniviral vector that contains the origin of adenoviral replication (in ITR) and the sequences for adenoviral packaging (pac⁺). This plasmid is designed to replicate and encapsidate in the presence of a helper virus. β-galactosidase was cloned in the *Not*I site as a reporter (plasmid 3).

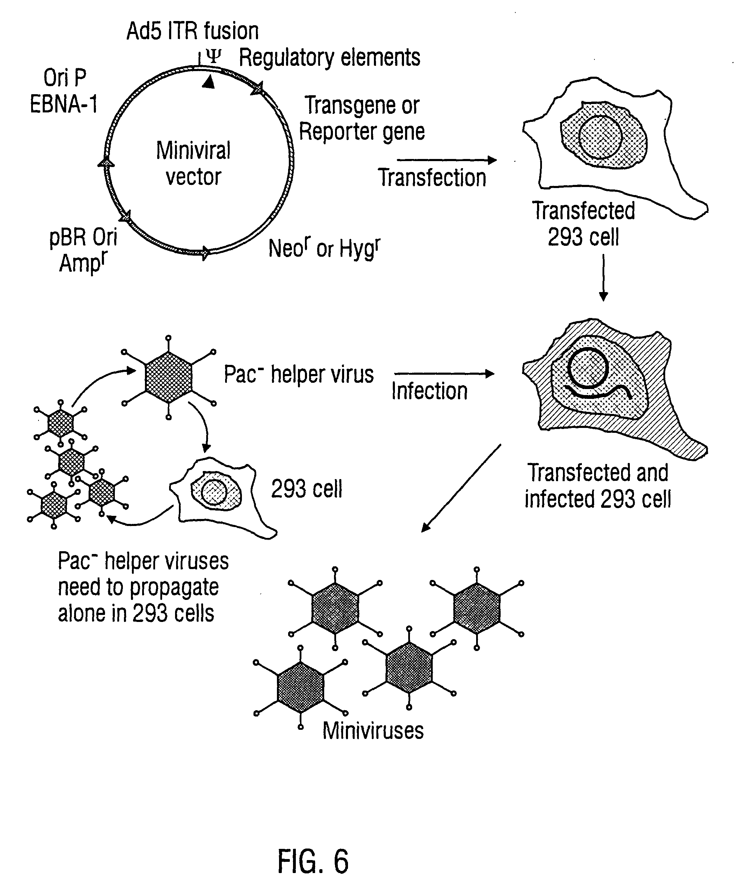

**FIG. 6.** To encapsidate the adenoviral vector, minivector DNA is transfected into E1-expressing cells with calcium-phosphate precipitation or by liposome-mediated gene transfer. After 24 hours, the transfected cells are further infected with the pac⁻ helper virus. After transfection and infection, the helper virus genome produces first the DNA-binding protein, terminal protein and polymerase, permitting efficient replication of both minivirus and helper virus genomes. Subsequently, structural proteins for capsid formation are produced. In competition for limited packaging factors, only the pac⁺ minivirus is packaged.

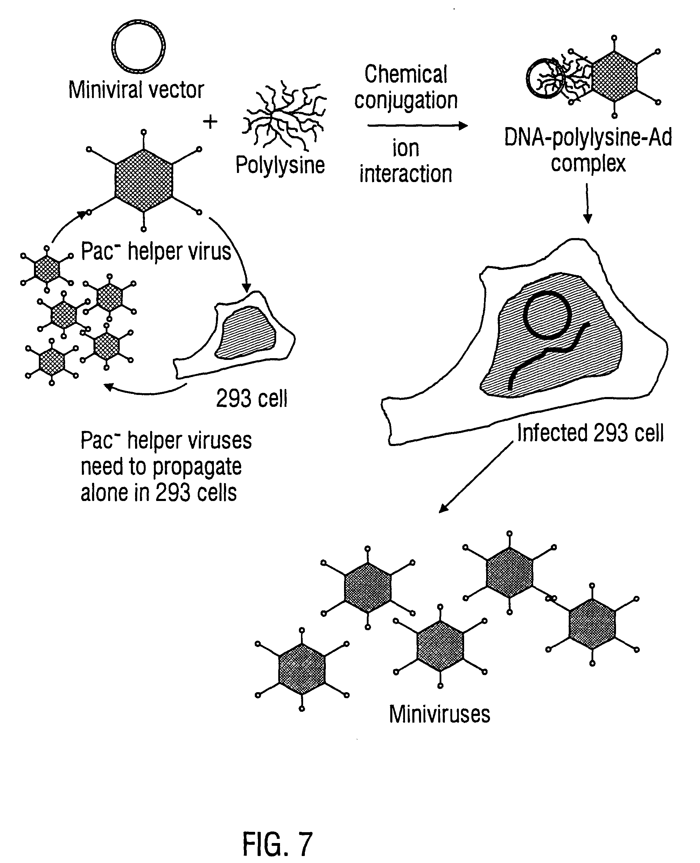

**FIG. 7.** As an alternative method to that described in FIG. 6, the minivector DNA will be bound to the pac-helper virus through polylysine and directly transduced into E1-expressing cells. This method may be more efficient than the method in FIG. 6 because the transduction efficiency is higher and the DNA stoichiometry between the minivector DNA and the helper viral DNA can be optimized *in vitro.* After delivery of the minivector and helper viral DNA into E1-expressing cells, the process of transcomplementation should be the same as that described in FIG. 6.

### DETAILED DESCRIPTION OF THE PREFERRED EMBODIMENTS

Gene therapy generally involves three principal elements: therapeutic genes, delivery systems and target cells. One of the urgent technical challenges in gene therapy technology is how to specifically deliver and controllably express the therapeutic genes in target cells *in vivo.* Currently available delivery system are limited in their ability to accomplish these goals (Mulligan, 1993), and there is a great demand for a new system with these capabilities.

The present invention is submitted to represent a significant advance in the genetic engineering of adenoviral vectors and their use in transfer of heterologous DNA into mammalian cells, particularly in the context of gene therapy. This new system will not only substantially increase the gene-delivery capacity of adenoviral vectors, but will also greatly improve their therapeutic potential, since the replacement of the viral genome eliminates the vector-borne cytotoxicity and the possibility of wild-type recombination events that are associated with the current Ad vector systems. Because the helper cell/helper virus system is capable of supporting a wide variety of mutations, the potential use for this system is extensive.

Replication of Ad mutants with deletions in different regions of the viral genome can be supported by helper viruses that provide the deleted gene products in *trans.* One example of this phenomenon involves the case of adenovirus/SV40 hybrid recombinants. Gluzman and Van Doren (1983) identified a recombinant that contained about 3500 base pairs from the left end of the Ad5 viral genome followed by 2.7 copies of the SV40 genome. This structure was repeated in the opposite orientation and, therefore, contained two Ad5 inverted terminal repeats and proper packaging signals. Other similar hybrids have been reported between host cell DNA and Ad12 (Deuring *et al.*, 1981). If the deletion of adenoviral sequences is small, the hybrids may be replicative. Otherwise, coinfection with a helper (wild-type) adenovirus is required for replication.

Another example of an adenoviral helper virus was reported by Challberg and Ketner (1981). They showed that Ad2 variants carrying large deletions can be complemented by a conditionally-defective virus. These conditionally-defective helper have a temperature-sensitive mutation outside of the region missing from the Ad2 variants. When grown together with Ad2 variants, the helpers provide the functions missing from the variants. The overall conclusion of Challberg and Ketner was that "it seems unlikely that complementing, helper viruses will prove to be generally useful," citing recombination and isolation problems.

A model system where helper viruses have been used successfully to support propagation of defective viral vectors is the Alphavirus expression system. Bredenbeek *et al.* (1993) report on the use of self-replicating replicons of Sindbis virus that carry and express heterologous genes. Though these RNAs are capable of replication within host cells following introduction as naked nucleic acids, *trans* complementation of virion structural sequences is needed to achieve packaging and release of infectious particles. In order to complement these defects, the authors provided a series of replication-defective helpers which provided the virion structural genes missing from the replicons. Various of these helpers were either packaging competent or packaging incompetent, the latter being useful in applications where virus spread is not desired. This system, although very useful for high level expression of protein, is not useful for gene therapy because of high cytopathogenicity due to inhibition of host cell protein synthesis.

### A. Adenovirus

Adenovirus is particularly suitable for use as a gene transfer vector because of its mid-sized DNA genome, ease of manipulation, high titer, wide target-cell range, and high infectivity. The roughly 36 kB viral genome is bounded by 100-200 base pair (bp) inverted terminal repeats (ITR), in which are contained *cis*-acting elements necessary for viral DNA replication and packaging. The early (E) and late (L) regions of the genome that contain different transcription units are divided by the onset of viral DNA replication.

The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression, and host cell shut off (Renan, 1990). The products of the late genes (L1, L2, L3, L4 and L5), including the majority of the viral capsid proteins, are expressed only after significant processing of a single primary transcript issued by the major late promoter (MLP). The MLP (located at 16.8 map units) is particularly efficient during the late phase of infection, and all the mRNAs issued from this promoter possess a 5' tripartite leader (TL) sequence which makes them preferred mRNAs for translation.

In order for adenovirus to be optimized for gene therapy, it is necessary to maximize the carrying capacity so that large segments of DNA can be included. It also is very desirable to reduce the toxicity and immunologic reaction associated with certain adenoviral products. The two goals are, to an extent, coterminous in that elimination of adenoviral genes serves both ends. By practice of the present invention, it is possible achieve both these goals while retaining the ability to manipulate the therapeutic constructs with relative ease.

The large displacement of DNA is possible because the *cis* elements required for viral DNA replication all are localized in the inverted terminal repeats (ITR) (100-200 bp) at either end of the linear viral genome. Plasmids containing ITR's can replicate in the presence of a non-defective adenovirus (Hay *et al*., 1984). Therefore, inclusion of these elements in an adenoviral vector should permit replication.

In addition, the packaging signal for viral encapsidation is localized between 194-385 bp (0.5-1.1 map units) at the left end of the viral genome (Hearing *et al*., 1987). This signal mimics the protein recognition site in bacteriophage λ DNA where a specific sequence close to the left end, but outside the cohesive end sequence, mediates the binding to proteins that are required for insertion of the DNA into the head structure. E1 substitution vectors of Ad have demonstrated that a 450 bp (0-1.25 map units) fragment at the left end of the viral genome could direct packaging in 293 cells (Levrero *et al.*, 1991).

Previously, it has been shown that certain regions of the adenoviral genome can be incorporated into the genome of mammalian cells and the genes encoded thereby expressed. These cell lines are capable of supporting the replication of an adenoviral vector that is deficient in the adenoviral function encoded by the cell line. There also have been reports of complementation of replication deficient adenoviral vectors by "helping" vectors, *e.g*., wild-type virus or conditionally defective mutants. It has not previously been possible to purify stocks of replication-deficient viruses away from helper viruses and concerns regarding "rescue" of wild-type viruses abound.

### B. An Adenovirus Helper System

The present invention is based, in part, on the observation that replication-deficient adenoviral vectors can be complemented, in *trans,* by helper virus. This observation alone did not permit isolation of the replication-deficient vectors, however, since the presence of helper virus, needed to provide replicative functions, would contaminate any preparation. Thus, an additional element was needed that would add specificity to the replication and/or packaging of the replication-deficient vector. That element derives from the packaging function of adenovirus.

It has been shown that a packaging signal for adenovirus exists in the left end of the conventional adenovirus map (Tibbetts, 1977). Later studies showed that a mutant with a deletion in the E1A (194-358 bp) region of the genome grew poorly even in a cell line that complemented the early (E1A) function (Hearing and Shenk, 1983). When a compensating adenoviral DNA (0-353 bp) was recombined into right end of the mutant, the virus was packaged normally. Further mutational analysis identified a short, repeated, position-dependent element in the left end of the Ad5 genome. One copy of the repeat was found to be sufficient for efficient packaging if present at either end of the genome, but not when moved towards the interior of the Ad5 DNA molecule (Hearing *et al*., 1987).

By using mutated versions of the packaging signal, it is possible to create helper viruses that are packaged with varying efficiencies. Typically, the mutations are point mutations or deletions. When helper viruses with low efficiency packaging are grown in helper cells, the virus is packaged, albeit at reduced rates compared to wild-type virus, thereby permitting propagation of the helper. When these helper viruses are grown in cells along with virus that contains wild-type packaging signals, however, the wild-type packaging signals are recognized preferentially over the mutated versions. Given a limiting amount of packaging factor, the virus containing the wild-type signals are packaged selectively when compared to the helpers. If the preference is great enough, stocks approaching homogeneity should be achieved.

### C. Cell Lines

Recombinant cell lines which express part of the adenoviral genome are described herein. These cells lines are capable of supporting replication of adenovirus recombinant vectors and helper viruses having defects in certain adenoviral genes, *i.e*., are "permissive" for growth of these viruses and vectors. The recombinant cell also is referred to as a helper cell because of the ability to complement defects in, and support replication of, replication-incompetent adenoviral vectors. The prototype for an adenoviral helper cell is the 293 cell line, which contains the adenoviral E1 region. 293 cells support the replication of adenoviral vectors lacking E1 functions by providing *in trans* the El-active elements necessary for replication.

Helper cells used herein are derived from a mammalian cell and, preferably, from a primate cell such as human embryonic kidney cell. Although various primate cells are preferred and human or even human embryonic kidney cells are most preferred, any type of cell that is capable of supporting replication of the virus would be acceptable.

Other cell types might include, but are not limited to Vero cells, CHO cells or any eukaryotic cells for which tissue culture techniques are established as long as the cells are adenovirus permissive. The term "adenovirus permissive" means that the adenovirus or adenoviral vector is able to complete the entire intracellular virus life cycle within the cellular environment.

The helper cell may be derived from an existing cell line, *e.g*., from a 293 cell line, or developed *de novo.* Such helper cells express the adenoviral genes necessary to complement *in trans* deletions in an adenoviral genome or which supports replication of an otherwise defective adenoviral vector, such as the E1, E2, E4, E5 and late functions. A particular portion of the adenovirus genome, the E1 region, has already been used to generate complementing cell lines. Whether integrated or episomal, portions of the adenovirus genome lacking a viral origin of replication, when introduced into a cell line, will not replicate even when the cell is superinfected with wild-type adenovirus. In addition, because the transcription of the major late unit is after viral DNA replication, the late functions of adenovirus cannot be expressed sufficiently from a cell line. Thus, the E2 regions, which overlap with late functions (L1-5), will be provided by helper viruses and not by the cell line. Typically, a cell line according to the present invention will express E1 and/or E4.

As used herein, the term "recombinant" cell is intended to refer to a cell into which a gene, such as a gene from the adenoviral genome or from another cell, has been introduced. Therefore, recombinant cells are distinguishable from naturally-occurring cells which do not contain a recombinantly-introduced gene. Recombinant cells are thus cells having a gene or genes introduced through "the hand of man."

Replication is determined by contacting a layer of uninfected cells, or cells infected with one or more helper viruses, with virus particles, followed by incubation of the cells. The formation of viral plaques, or cell free areas in the cell layer, is the result of cell lysis caused by the expression of certain viral products. Cell lysis is indicative of viral replication.

### D. Vectors

The virion particle of the present invention contains an adenovirus vector construct in which at least a portion of the E1 and E3 regions of the virus are deleted, along with at least portion of the E4 and/or E2 regions. In an alternative embodiment, the defects in the E1 and E3 regions may not be deletions but point mutations rendering the "early" gene products inactive or preventing their synthesis entirely. Examples of preferred embodiments provided herein make use of the adenovirus 5 serotype (Ad5) genome. It is understood, however, that other serotypes such as the adenovirus type 2 (Ad2) genome, for example, would also function in the practice of the invention.

Three benefits arise from various forms of adenovirus mutants. Where a mutation simply renders a protein non-functional, the ability of the virus to replicate once administered to a patient is eliminated, thus lessening the chance for pathogenic effects. If the protein mutation also results in the absence of a protein product, an additional benefit in terms of lower toxicity is realized. Finally, if the adenovirus mutant lacks some or all of the gene segment encoding the protein, the apathogenic and non-toxic phenotype is achieved along with increased capacity to carry foreign genes. Thus, an adenovirus mutant lacking at least a portion of its coding sequence is preferred.

The invention also can be described as a virion particle containing an adenovirus vector construct comprising at least about 350 base pairs of the left ITR region of the Ad5 genome, up to about 35 kB of heterologous DNA, and at least about 100 base pairs of the right ITR region of the Ad5 genome (see FIG. 1), the virion particle being modified to incorporate a cell targeting agent that is encoded by a gene inserted into the vector construct. Corresponding regions of other serotypes, such as the adenovirus type 2 genome, can be used as well. In its most preferred embodiment, the left and right ITR regions will flank the heterologous DNA and contain said heterologous DNA between them. Any arrangement of the viral and heterologous DNA that permits replication and encapsidation is acceptable, however, and is included as a part of the present invention.

A vector containing a 5.5 kB insert inserted in place of the E1 and E3 regions and including the additional 2 kB that the virus can package was known in the art. In the virion particle of the present invention, more than 36 kB of heterologous DNA can be contained in the vector, depending on the size of the deletion. Different vectors lacking 10, 15, 20, 30 and 35 kB of adenoviral sequences are contemplated. The present invention makes possible, for example, deletion of the E1, E2, E3, E4, L1, L2, L3, L4 and L5 regions, or any combination of these regions, and replacement of the deleted regions with heterologous DNA.

The adenovirus vector construct must therefore replicate in a helper cell with the aid of one or more helper viruses. In order for replication to occur, the vector must encode all of the necessary *cis*-acting elements needed for replication of the vector DNA, including those required for initiation of genome replication and for packaging of the replicated DNA into the viral capsid, provided that the remaining *trans* elements are supplied by the helper cell and the helper virus.

In the context of the adenovirus vector, the term heterologous DNA is meant to include DNA derived from a source other than the adenovirus genome which provides the backbone of the vector. This heterologous DNA may be derived from a prokaryotic or a eukaryotic source such as a bacterium, a virus, a yeast, a plant or even an animal. The heterologous DNA may also be derived from more than one source. For instance, a regulatory sequence may be derived from a virus and may control the expression of a structural gene from a different source, such as a mammal.

Regulatory elements include promoters. Preferred promoters are viral promoters such as the adenovirus major later promoter, SV40 late promoter from simian virus 40, the Baculovirus polyhedron enhancer/promoter element, Herpes Simplex Virus thymidine kinase (HSV *tk*), the immediate early promoter from cytomegalovirus (CMV) and various retroviral promoters including LTR elements. The elements are operably linked to a gene, the expression of which is desired. By "operably linked," it is meant that the regulatory element is positioned, relative to a coding sequence, such that expression of that coding sequences is effected or enhanced by that element.

The promoters and enhancers preferably employed will be those that control the transcription of protein encoding genes in mammalian cells and may be composed of multiple genetic elements. The term promoter includes that group of transcriptional control modules clustered around the initiation site for RNA polymerase II. Promoters are believed to be composed of discrete functional modules, each comprising approximately 7-20 bp of DNA, and containing one or more recognition sites for transcriptional activator proteins. At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between some elements is flexible, so that promoter function is preserved when elements are inverted or moved relative to one another. Depending on the promoter, it appears that individual elements can function either cooperatively or independently to activate transcription.

The promoter further may be characterized as an inducible promoter. An inducible promoter is a promoter which is inactive or exhibits low activity except in the presence of an inducer substance. Some examples of inducible promoters that may possibly be included as a part of the present invention include, but are not limited to, MT II, MMTV (mouse mammary tumor virus), Collagenase, Stromelysin, SV40, Murine MX Gene, α-2-Macroglobulin, MHC Class I Gene H-2kb, HSP70, Proliferin, Tumor Necrosis Factor or Thyroid Stimulating Hormone α Gene. It is understood that any inducible promoter may be used in the practice of the invention.

Another type of promoter that may be included within the heterologous DNA is a tissue specific promoter. A tissue specific promoter is a promoter that preferentially is active in a cell of a particular type, such as in liver, muscle, endothelia and the like. Some examples of tissue specific promoters that may be used in the practice of the invention include the albumin promoter, expressed in the liver, or the surfactin promoter, expressed in the lung. The muscle-specific creatine kinase enhancer, in combination with the human cytomegalovirus immediate early promoter, is a preferred construct for expression in muscle tissue, for example.

The heterologous DNA may also comprise an enhancer, also operably linked to the gene of interest. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Aside from this operational distinction, enhancers and promoters are very similar. They have the same general function of activating transcription in the cell and often have overlapping, contiguous and seemingly similar modular organization. Taken together, these considerations suggest that enhancers and promoters are homologous entities and that the transcriptional activator proteins bound to these sequences may interact with the cellular transcriptional machinery in fundamentally the same way. It is understood that any such promoter, enhancer or promoter/enhancer combination may be included in the heterologous DNA of the adenoviral vector to control expression of the heterologous gene regions.

The heterologous DNA may include more than one structural gene under the control of the same or different promoters. The heterologous DNA may also include ribosome binding sites and polyadenylation sites or any other elements necessary for the expression of the DNA in a eukaryotic or a mammalian cell. These elements, along with appropriate promoter and enhancer elements, are combined into vector constructs by methods well known and routinely practiced in the art such as restriction enzyme digestion followed by DNA ligase directed splicing of the various genetic elements. The heterologous DNA may include regions from other viruses that can confer specific properties on the construct such as integration capability or the ability to replicate in the presence of various other viruses.

### E. Targeting

The invention provides a virion particle containing a packaged adenovirus vector construct. The virion particle is capable of infecting cells as a means of introducing the vector DNA into cells wherein the heterologous DNA is expressed. Techniques for the replication of adenoviral vectors and infection cf target cells are well known and routinely practiced in the art.

The virion capsid of the virion particle according to the invention is modified to incorporate a cell targeting agent such as an antibody or a cell receptor recognition peptide to target the virions to particular cells. Such targeting agents are encoded by a gene inserted into the vector construct.

More specifically, the targeting mechanism may include insertion, into the viral capsid genes, DNA fragments that encoding a binding site peptide or a ligand peptide that would serve to target the virion to a particular type of cell or to cells expressing certain surface proteins. One particularly example would be the expression of the Fc binding region from protein A on the capsid surface. Such altered viruses could then be treated with an antibody specific for a certain cell or tissue type. The virus capsid would bind to the antibodies and be directed to cells bearing the antigen recognized by the antibodies.

Another example of cell targeting is the expression of a ligand binding site on the surface of the virus. In this example, the virus would bind directly to the ligand on the targeted cell or tissue surface. Another example of targeting would be the expression of an cell-specific epitope on the virus capsid. In this technique, the virus particles would be treated with monoclonal antibodies to the epitope. Because the monoclonal antibodies are bivalent, the antibodies would retain a free binding arm for targeting of the virus particles to the target cell or tissue. The foregoing discussion of targeting should not be read as precluding the direct inoculation of virus to a target area. A preparation containing the virus particles can be injected into a local area, such as an organ or into a tumor, or applied thereto following surgical exposure.

### F. Helper Virus

A helper virus is defined as an adenovirus that can complement the replication and packaging of the adenovirus vector construct contained in the virion particle of the invention.

Helper vectors require the same *cis*-acting sequences as the adenoviral vectors, namely, an origin of replication and a packaging signal. Preferably, a helper virus also contains a mutation in the adenovirus packaging signal that causes it to be utilized less efficiently than the wild-type packaging signal, although it still is utilized to an extent that packaging will occur in the absence of competing, wild-type signals. Like the adenoviral vectors, the helper virus will need to be propagated on a helper cell line that compensates for its defects. Usually, the defects will include deletions in the E1 and/or E2 regions of the helper virus genome. Also, the non-essential E3 region may be removed. A list of some possible combinations is provided in the following table.

**TABLE 1:**

| **PHENOTYPES OF VARIOUS COMPONENTS** | | |
|---|---|---|
| VECTOR | HELPER CELL | HELPER VIRUS |
| E4 | E1 | E2, L1-L5 |
| E4 | - | E1, E2, L1-L5 |
| - | E1 | E2, E4, L1-L5 |
| - | E1, E4 | E2, L1-L5 |
| - | E4 | E1, E2, L1-L5 |
| - signifies absence of functional E1-E5 and L1-L5 products | | |

One of the advantages provided by the present system is the greatly reduced possibility that the adenoviral vector, through homologous recombination, will reacquire a wild-type genome, *i.e*., be rescued, at the cost of eliminating the heterologous DNA sequences. By using three different genetic entities (helper cell, helper virus and adenoviral vector) rather than two (helper cell and adenoviral vector; helper virus and adenoviral vector), it becomes impossible for a single recombination event to rescue wild-type virus.

### G. Method of Expressing Heterologous Genes

While the virion particles provided by the present invention are particularly useful in gene therapy, they also are quite useful in *in vitro* methods for the manipulation and expression of genes in other contexts. Such methods involve the use of an adenoviral vector construct containing heterologous DNA encoding the foreign gene and means for its expression, replicating the adenoviral vector construct in an appropriate helper cell with an appropriate helper virus, obtaining virion particles produced therefrom and infecting mammalian cells with the virion particles. The foreign gene could be, for example, a cystic fibrosis gene, an interleukin or antiviral product. It also is contemplated that the gene may encode a toxin or a gene that otherwise renders a cell susceptible to further treatment with a pharmaceutical agent.

Another example of a disease for which a large capacity vector might be effective is Duchenne muscular dystrophy (DMD), a lethal, X-linked degenerative disorder of muscle, which affects about 1 in 35,000 newborn males. DMD is caused by a deficiency of dystrophin (Zubrzycka-Gaarn *et al*., 1988), a 427 kD protein encoded by a 14 kB transcript (Koenig *et al*., 1987). A possible therapy for this disease would be the restoration of dystrophin function by insertion and expression of the dystrophin gene in the patient's muscles (Blau, 1993; *Cox et al.,* 1993). This therapy would require a vector that could efficiently deliver the 14 kB cDNA into muscle cells and specifically express the DMD protein in the muscle cells. Unfortunately, at the time of this disclosure, there is no vector system available which is capable of delivering more than 7.5 kB of DNA to be expressed in a specific tissue.

The foreign gene to be expressed, as described in the preceding paragraph, may be of any origin, for example, a bacterium, a yeast, a plant, an animal or even a human gene. Preferably, the foreign gene is configured as a complementary DNA (cDNA). In some instances, however, it may prove advantageous to use a genomic DNA clone where, for example, introns contained therein provide some additional benefit. It also is contemplated that antisense constructs, specifically designed to inhibit expression of a particular gene, will be useful.

As discussed above, a variety of regulatory elements including promoters, enhancers, polyadenylation sites, *etc.,* may be included in the foreign gene. The adenovirus vector construct contains a deletion in the E1 and E3 region of the genome and at least one other adenovirus "early" or "late" region, and the foreign gene is inserted in place of the missing adenoviral sequences.

Virus particles produced by the replicating viral vector in the helper cell, along with the helper virus, can be obtained by any acceptable means. Such means would include filtration, centrifugation. Plaque purification, by direct isolation of virus from lysed cells also is possible. All such methods of obtaining virion particles and infecting mammalian cells with such particles are well known to those of skill in the art.

A first step in expressing a foreign gene is the development of adenoviral helper viruses. The general scheme for generating helper viruses is as follows. First, a part of the adenoviral genome is inserted into a standard cloning vector. A deletion in that region is then engineered. Following amplification of the deletion construct, the adenoviral fragment is excised and cotransfected with adenoviral genomic DNA in a cell line expressing the "early" functions deleted from the adenoviral fragment. Following recombination, adenoviral genomic DNA lacking the deleted sequences are isolated. This process can be repeated to incorporate additional deletions so long as cell lines are available that can complement the increasing number of defective functions.

The adenoviral genomic DNA that receives the deletion fragments may contain a mutation in the packaging signal (pac⁻). Because the recombination of deletions into the adenoviral genome is accomplished in the absence of other adenoviruses, however, the mutated packaging signal is sufficient to permit encapsidation. Subsequent propagation of this virus with vectors containing wild-type packaging signals will result in preferential encapsidation of the vectors.

Also important is the development of Ad helper cell lines. These cells lines are the stably transfected, or "transformed," with DNA from adenovirus. The DNA may be integrated or maintained as episomal fragments of Ad sequences. These cell lines are designed to express different sets of Ad proteins and can be used to generate and propagate different Ad vectors.

Another step in the helper virus system is the construction of adenoviral vectors. Ad vectors can be generated in two basic fashions. First, a region of the adenoviral genome can be inserted into a standard cloning vector. Next, a deletion is engineered into the adenovirus insert and the deleted sequences replaced with a heterologous DNA. Cotransfection of the Ad-hetDNA-Ad insert with adenoviral genomic DNA will result in recombination of the heterologous DNA, by virtue of the flanking Ad sequences, into the cotransfected adenoviral genomic DNA. If the host cells do not compensate for the adenoviral functions missing from the new recombinant, the cells can be superinfected with helper viruses.

An alternative method for generating Ad vectors would be the generation, *in vitro*, of the entire Ad sequences. Using restriction enzymes and DNA ligase, it is possible to clone directly the necessary *cis*-acting sequences from adenovirus to the heterologous DNA. This construct can then be transfected into helper cells, optionally infected with helper virus, for the purposes of replication and encapsidation. In this context, it may be helpful to generate an adenoviral vector containing only the packaging signal, origin of replication and a multipurpose cloning site for the insertion of heterologous DNA. In a preferred embodiment, this starting vector also would contain an excisable marker gene.

In any of the preceding or following discussion, the term transfecting should be understood as including an type of gene transfer methodology including calcium-phosphate precipitation, protoplast fusion, lipofection, cation-facilitated DNA (*e.g.,* polylysine) transduction or any other equivalent method. Together, these terms are deemed equivalent the phrase "importing nucleic acid."

For example, technology is available to conjugate naked nucleic acids to polycations, which conjugates are taken up by cells brought in contact with such conjugates. In certain embodiments, it also is desirable to include an agent capable of disrupting lysosomes in which the conjugate is taken up. A preferred lysosomal disruption agent is adenovirus itself. The adenovirus can be wild-type adenovirus, adenovirus containing a defective genome or empty adenovirus particles. An example of the approach is illustrated in FIG. 7.

### EXAMPLES

The following examples are included.

### Example 1: Construction of a Packaging Defective Adeno-Helper Virus

A helper virus was generated by recombination of overlapping sequences of a shuttle vector and cloned fragments of the adenovirus genome. The *Eco*RI*-Cla*I small fragment from pXCJ.2 (Spessot *et al*., 1989) was subcloned into the respective sites of pBSKS (Stratagene) to generate pBSleft-end. A 1.7 kB fragment from nucleotides 4021 to 5785 of Ad5 was synthesized by PCR using pJM17 (McGrory *et al*., 1988) as a template using primers 5'-CCATCGATGCGGTTTAAAACATAAAT-3' (*Cla*I site underlined) and 5'-CCGCGGAACACCGCTCGAGGAC-3'. pRA was generated by inserting the PCR-generated fragments into *Cla*I-*Xho*I sites of pBSleft-end. Subsequent cleavage of pRA with *Sgra*I (nucleotide 188) and *Cla*I (nucleotide 450), effectively removing the wild-type packaging signal. The corresponding *Sgra*I*-Cla*I fragment from pE1A-10/28, containing a double-deletion in the Ad5 packaging signal (Grable and Hearing, 1990) was inserted into pRA. pJM17 and pRApac⁻ were cotransfected into 293 cells. Virus is then purified and cloned by limiting dilution on 293 cells, after which pac⁻ helper is identified.

### Example 2: Construction of a Replication Deficient Adenovirus Vector

By fusing the inverted terminal repeats (ITRs) of adenovirus with a prokaryotic origin of replication, a replicable adenovirus vector can be constructed that also may be propagated in bacteria. Such a vector has been generated by inserting the ITR fusion region of pAB17 (*Bsa*AI site at 35,771 to *Sac*II site at 358 (nucleotide nos. from Ad5) into the *XbaI* site of pREP9 (Invitrogen). This insert contains the wild-type packaging signal at nucleotides 194-358. As a reporter, the β-gal gene was subcloned as a *Not*I-*Not*I fragment from pTKβ (Clontech) into the *Not*I site following the Rous Sarcoma Virus promoter of pREP9.

### Example 3: Expression of Heterologous Peptide in Mammalian Cells

p53 is excised from pEC53 (Zhang *et al.* 1994) and inserted into the *Not*I site of the vector described in example. The plasmid is introduced into 293 cells by Dotap-mediated transfection. *Id.* After 24 hours, cells are infected with the helper virus described in Example 1 in complete medium. After cytopathic effects appear, virus is harvested by three freeze-thaw cycles and virus purified by CsCl density gradients. *Id*. Purified vector is stored at -80°C in buffered 10% glycerol.

### Example 4: Administration of a Therapeutic Vector to a Patient In Vivo

Large scale production of viral vector described in Example 3 will be undertaken and each batch evaluated for purity and homogeneity. Virus stocks can be stored at titers of 10¹¹ pfu/ml at -80°C. Patients with advanced (stage III) inoperable adenocarcinoma are selected for possible treatment and the tumors screened for p53 status. Those patients having tumors with deleted or mutated p53 are further selected for treatment. By fibroscopy, as much tumor mass as possible is removed and a fibroscopic-guided needle is used to inject vector at 0.1 ml volumes (10¹⁰ pfu) at 4-6 sites in the remaining tumor mass. Patients are monitored daily for systemic inflammation. After about 1 week, the tumor is biopsied to assess p53 expression and vector presence. Pharyngeal mucosa, urine and stool samples are taken in order to assess for possible adventious virus shedding.

### REFERENCES

Berkner, K.L. 1988. Development of adenovirus vectors for the expression of heterologous genes. *BioTechniques* **6**: 616-629.
Blau, H.M. 1993. Muscular dystrophy: Muscling in on gene therapy. *Nature* **364**: 673-675.
Bett, A.J., W. Haddara, L. Pzevec and F.L. Graham. 1994. An efficient flexible system for construction of adenovirus vector with insertions or deletions in early regions 1 and 3. *Proc. Nat'l Acad. Sci. USA* **91**: 8802-8806.
Boshart, J., F. Weber, G. Jahn, K. Dorsch-Hasler, B. Fleckenstein, and W. Schaffner. 1985. A very strong enhancer is located upstream of an immediate early gene of human cytomegalovirus. *Cell* **41**: 521-530.
Bredenbeek, P.J., I. Frolov, C.M. Rice, and S. Schlesinger. 1993. Sindbis virus expression vectors: packaging of RNA replicons by using defective helper RNAs. *J. Viol.* **67**: 6439-6446.
Bridge, E., S. Medghalchi, S. Ubol, M. Leesong, and G. Ketner. 1993. Adenovirus early region 4 and viral DNA synthesis. *Virology* **193**: 7994-801.
Challberg and Ketner. 1981. Deletion of mutants of Adenovirus 2: isolation and initial characterization of virus carrying mutations near the right end of the viral genome. *Virology* **114**: 196-209.
Chiao, P.J., F.Z. Bischoff, L.C. Strong, and M.A. Tainsky. 1990. The current state of oncogenes and cancer: experimental approaches for analyzing oncogenetic events in human cancer. *Cancer Metastasis Rev*. **9**: 63-80.
Couch, R.B., R.M. Chanock, T.R. Cate, D.J. Lang, V. Knight, and R.J. Huebner. 1963. Immunization with types 4 and 7 adenovirus by selective infection of the intestinal tract. *Am. Rev. Resp. Dis*. **88**: 394-403.
Cox, G.A., N.M. Cole, K. Matsumura, S.F. Phelps, S.D. Hauschka, K.P. Campbell, J.A. Faulkner, and J.S. Chamberlain. 1993. Overexpression of dystrophin in transgenic mdx mice eliminates dystrophic symptoms without toxicity. *Nature* **364**: 725-729.
Dai, Y., M. Roman, R.K. Naviaux, and I.M. Verma. 1992. Gene therapy via primary myoblasts: long-term expression of factor IX protein following transplantation *in vivo. Proc. Natl. Acad. Sci. USA* **89**: 10892-10895.
Deluca, N.A., A.M. McCarthy, and P.A. Schaffer. 1985. Isolation and characterization of deletion mutants of herpes simplex virus type 1 in the gene encoding immediate-early regulatory protein ICP4. *J. Virol.* **56**: 558-570.
Deuring, R., Klotz, G., and Doerfler, W., 1981, An unusual symmetric recombinant between adenovirus type 12 DNA and human cell DNA, *Proc. Natl. Acad. Sci. U.S.A.* **78**: 3142-3146
Friedmann, T. 1989. Progress toward human gene therapy. *Science* **244**: 1275-1281.
Friedmann, T. 1992. Gene therapy of cancer through restoration of tumor-suppressor functions? *CANCER* **70** (Suppl): 18101-1817.
Geller, A.I. 1993. Herpesviruses: expression of genes in postmitotic brain cells. *Curr. Opin. Genet. Devel.* **3**: 81-85.
Geller, A.I., and H.J. Federoff. 1991. The use of HSV-1 vectors to introduce heterologous genes into neurons: implications for gene therapy. In: *Human Gene Transfer,* Eds, O. Cohen-Haguenauer and M. Boiron, pp. 63-73, Editions John Libbey Eurotext, France.
Ghosh-Choudhury, G., Y. Haj-Ahmad, and F.L. Graham. 1987. Protein IX, a minor component of the human adenovirus capsid, is essential for the packaging of full-length genomes. *EMBO J.* **6**: 1733-1739.
Glorioso, J.G., W.F. Goins, and D.J. Fink. 1992. Herpes simplex virus-based vectors. *Semin. Virol.* **3**: 265-276.
Gluzman, Y., and Van Doren, K. 1982. Palindromic Adenovirus Type 5-Simian Virus 40 Hybrid. *J. Virol.* **45**:91-103.
Goebel, S.J., G.P. Johnson, M.E. Perkus, S.W. Davis, J.P. Winslow, and E. Paoletti. 1990. The complete DNA sequence of vaccinia virus. *Virology* **179**: 247-266.
Gomez-Foix, A.M., W.S. Coats, S. Baque, T. Alam, R.D. Gerard, and C.B. Newgard. 1992. Adenovirus-mediated transfer of the muscle glycogen phosphorylase gene into hepatocytes confers altered regulation of glycogen. *J. Biol. Chem.* **267**: 25129-25134.
Graham, F.L., and L. Prevec. 1991. Manipulation of adenovirus vectors. In: Methods in Molecular Biology (Vol. 7), *Gene Transfer and Expression Protocols,* ed. E.J. Murray, The Humana Press Inc., Clifton, NJ.
Graham, F.L., J. Smiley, W.C. Russell, and R. Nairn. 1977. Characteristics of a human cell line transformed by DNA from human adenovirus type 5. *J. Gen. Virol.* **36**: 59-72.
Graham, F.L. 1984. Covalently closed circles of human adenovirus DNA are infectious. *EMBO J.* **3**: 3917-2922.
Graham, F.L., and L. Prevec. 1992. Adenovirus-based expression vectors and recombinant vaccines. *Biotechnology* **20**: 363-390.
Grunhaus, A., and M.S. Horwitz. 1992. Adenoviruses as cloning vectors. *Semin. Virol.* **3**: 237-252.
Haj-Ahmad, Y., and F.L. Graham. 1986. Development of a helper-independent human adenovirus vector and its use in the transfer of the herpes simplex virus thymidine kinase gene. *J. Viol.* **57**: 2267-274.
Hammarskjold, J.-L., G. Winberg, E. Norrby, and G. Wadell. 1977. Isolation of incomplete adenovirus 16 particles containing viral and host cell DNA. *Virology* **82**: 449-461.
Hay, R.T., N.D. Stow and I.M. McDougall. Replication of Adenovirus Mini-Chromosomes. 1984. *J. Mol. Biol.* **175**:493-510.
Hearing, P., R.J. Samulsi, W.L. Wishart, and T. Shenk. 1987. Identification of a repeated sequence element required for efficient encapsidation of the adenovirus type 5 genome. *J. Virol.* **67**: 2555-2558.
Hearing, P., and Shenk, T., 1983, Functional analysis of the nucleotide sequence surrounding the cap site for adenovirus type 5 region Ela messenger RNAs. *J. Mol. Biol*. **167**:809-822.
Hermonat, P.L., and N. Muzyczka. 1984. Use of adeno-associated virus as a mammalian DNA cloning vector: transduction of neomycin resistance into mammalian tissue culture cells. *Proc. Natl. Acad. Sci. USA* **81**: 6466-6470.
Herz, J., and R.D. Gerard. 1993. Adenovirus-mediated transfer of low density lipoprotein receptor gene acutely accelerates cholesterol clearance in normal mice. *Proc. Natl. Acad. Sci. USA* **90**: 2812-2816.
Horwitz, M.S. 1990. Adenoviridae and their replication. In: *Virology* (2nd Edn), eds. B.N. Field, D.M. Knipe, R.M. Chanock, J.L. Melnick, M.S. Hirsch, T.P. Monath, B. Roizman. pp. 1679-1721. Raven Press, New York.
Jones, N., and T. Shenk. 1978. Isolation of deletion and substitution mutants of adenovirus type 5. *Cell* **13**:181-188.
Knipe, D.M., W. T. Ruyechan, B. Roizman, and I.W. Halliburton. 1978. Molecular genetics of herpes simplex virus: demonstration of regions of obligatory and nonobligatory identity within diploid regions of the genome by sequence replacement and insertion. *Proc. Natl. Acad. Sci. USA* **75**: 3896-3900.
Koenig, M., E.P. Hoffman, C.J. Bertelson, A.P. Monaco, C. Feener, and L.M. Kunkel. 1987. Complete cloning of the Duchenne muscular dystrophy (DMD) cDNA and preliminary genomic organization of the DMD gene in normal and affected individuals. *Cell* **50**: 509-517.
Kriegler, M.P. 1990. *Gene Transfer and Expression: A Laboratory Manual.* pp. 23-61. Stockton Press, New York.
Larsen, S.H. 1982. Evolutionary variants of mouse adenovirus containing cellular DNA sequences. *Virology* **116**: 573-580.
Le Gal La Salle, G., J.J. Robert, S. Berrard, V. Ridoux, L.D. Stratford-Perricaudet, M. Perricaudet, and J. Mallet. 1993. An adenovirus vector for gene transfer into neurons and glia in the brain. *Science* **259**: 988-990.
Lebkowski, J.S., M.M. McNally, T.B. Okarma, and L.B. Lerch. 1988. Adeno-associated virus: a vector system for efficient introduction and integration of DNA into a variety of mammalian cell types. *Mol. Cell Biol.* **8**: 3988-3996.
Levine, A.J. 1990. Tumor suppressor genes. *BioEssays* **12**: 60-66.
Levrero, M., V. Barban, S. Manteca, A. Ballay, C. Balsamo, M.L. Avantaggiati, G. Natoli, H. Skellekens, P. Tiollais, and M. Perricaudet. 1991. Defective and nondefective adenovirus vectors for expressing foreign genes *in vitro* and *in vivo. Gene* **101:** 195-202.
Lotze, M.T., J.T. Rubin, and H.J. Zeh. 1992. New biologic agents come to bat for cancer therapy. *Curr. Opin. Oncol.* **4**: 1116-1123.
Majors, J.E. 1992. Retroviral vector-strategies and applications. *Semin. Virol.* **3**: 285-295.
McGrory, W.J., D. S. Bautista, and F.L. Graham. 1988. A simple technique for the rescue of early region 1 mutations into infectious human adenovirus type 5. *Virology* **163**: 614-617.
Miller, A.D., and G.J. Rosman. 1989. Improved retroviral vectors for gene transfer and expression. *BioTechniques* **7**: 980-990.
Miller, A.D. 1992. Human gene therapy comes of age. *Nature* **357**: 455-460.
Morgan, R.A., L. Couture, O. Elroy-Stein, J. Ragheb, B. Moss, and W.F. Anderson. 1992. Retroviral vectors containing putative internal ribosome entry sites: development of a polycistronic gene transfer system and applications to human gene therapy. *Nucleic Acids Res.* **20**: 1293-1299.
Morgenstern, J.P., and H. Land. 1991. Choice and manipulation of retroviral vectors. In: *Methods in Molecular Biology* (*Vol. 7)*, *Gene Transfer and Expression Protocols,* ed. E.J. Murray, The Humana Press Inc., Clifton, NJ.
Moss, B. 1992. Poxviruses as eukaryotic expression vectors. *Semin*. *Virol.* **3**: 277-283.
Moss, B. 1991. Vaccinia virus: a tool for research and vaccine development. *Science* **252**: 1662-1667.
Mulligan, R.C. 1993. The basic science of gene therapy. *Science* **260**: 9260-932.
Mullis, K., U.S. Patent 4,683,202,
Pardoll, D. 1992. Immunotherapy with cytokine gene-transduced tumor: the next wave in gene therapy for cancer. *Curr. Opin. Oncol.* **4**: 1124-1129.
Ragot, T., N. Vincent, P. Chafey, E. Vigne, H. Gilgenkrantz, D. Couton, J. Cartaud, P. Briand, J.-C. Kaplan, M. Perricaudet, and A. Kahn. 1993. Efficient adenovirus-mediated transfer of a human minidystrophin gene to skeletal muscle of mdx mice. *Nature* **361**: 647-650.
Renan, M.J. 1990. Cancer genes: current status, future prospects, and applicants in radiotherapy/oncology. *Radiother. Oncol.* **19**: 197-218.
Rich, D.P., L.A. Couture, L.M. Cardoza, V.M. Guiggio, D. Armentano, P.C. Espino, K.Hehir, M.J. Welsh, A.E. Smith, and R.J. Gregory. 1993. Development and analysis of recombinant adenoviruses for gene therapy of cystic fibrosis. *Hum. Gene Ther.* **4**: 461-476.
Roizman, R., and A.E. Sears. 1990. Herpes simplex viruses and their replication, In: *Virology,* (2nd ed), eds. B.N. Field, D.M. Knipe, R.M. Chanock, J.L. Melnick, M.S. Hirsch, T.P. Monath, B. Roizman. pp. 1795-1841. Raven Press, New York.
Rosenfeld, M.A., K. Yoshimura, B.C. Trapnell, K. Yoneyama, E.R. Rosenthal, W. Dalemans, M. Fukayama, J. Bargon, L.E. Stier, L. Stratford-Perricaudet, M. Perricaudet, W. B. Guggino, A. Pavirani, J.-P. Lecocq, and R.G. Crystal. 1992. *In vivo* transfer of the human cystic fibrosis transmembrane conductance regulator gene to the airway epithelium. *Cell* **68**: 143-155.
Rosenfeld, M.A., W. Siegfried, K. Yoshimura, K. Yoneyama, M. Fukayama, L.E. Stier, P.K. Paakko, P. Gilardi, L. Stratford-Perricaudet, M. Perricaudet, S. Jallat, A. Pavirani, J.-P. Lecocq, and R.G. Crystal. 1991. Adenovirus-mediated transfer of a recombinant α1-antitrypsin gene to the lung epithelium *in vivo. Science* **252**: 431-434.
Sambrook *et al.* (1989). *Molecular cloning: A laboratory manual.* Cold Spring Harbor Laboratory. Cold Spring Harbor, NY.
Samulski, R.J., L.-S. Chang, and T. Shenk. 1989. Helper-free stock of recombinant adeno-associated viruses: normal integration does not require viral gene expression. *J. Virol.* **63**: 3822-3828.
Smith, G.L., and B. Moss. 1983. Infectious poxvirus vectors have capacity for at least 25,000 base pairs of foreign DNA. *Gene* **25**: 21-28.
Spector, D.J., D.N. Halbert, H. Raskas. 1980. Regulation of integrated adenovirus sequences during adenovirus infection of transformed cells. *J. Virol.* **36**: 860-871.
Spessot, R., K. Inchley, T.M. Hupel, and S. Bacchetti. 1989. Cloning of the herpes simplex virus ICP4 gene in an adenovirus vector: effects on adenovirus gene expression and replication. *Virology*. **168**: 378-387.
Stratford-Perricaudet, L.D., M. perricaudet, and P. Briand. 1990. Evaluation of the transfer and expression in mice of an enzyme-encoding gene using a human adenovirus vector. *Hum. Gene Ther.* **1**: 241-256.
Stratford-Perricaudet, L., and M. Perricaudet. 1991. Gene transfer into animals: the promise of adenovirus. p. 51-61, In: *Human Gene Transfer,* Eds, O. Cohen-Haguenauer and M. Boiron, Editions John Libbey Eurotext, France.
Su, W., T. Middleton, B. Sugden, and H. Echols. 1991. DNA looping between the origin of replication of Epstein-Barr virus and its enhancer site: stabilization of an origin complex with Epstein-Barr nuclear antigen I. *Proc. Natl. Acad. Sci. USA* **88**: 10870-10874.
Sugimura, T., M. Terada, J. Yokota, S. Hirohashi, and K. Wakabayashi. 1992. Multiple genetic alterations in human carcinogenesis. *Environ. Health Perspect.* **98**: 5-12.
Tibbetts, C. 1977. Viral DNA sequences from incomplete particles of human adenovirus type 7. *Cell.* **12**: 243-249.
Top, F.H., Jr., El.L. Buescher, W.H. Bancroft, and P.K. Russell. 1971. Immunization with live types 7 and 4 adenovirus vaccines. II. Antibody response and protective effect against acute respiratory disease due to adenovirus type 7. *J. Infect. Dis.* **124**: 155-160.
Walsh, C.E., J.M. Liu, X. Xiao, N.S. Young, A.W. Nienhuis, and R.J. Samulski. 1992. Regulated high level expression of a human τ-globin gene introduced into erythroid cells by an adeno-associated virus vector. *Proc. Natl. Acad. Sci. USA* **89**: 7257-7261.
Weinberg, D.H., and G. Ketner. 1983. A cell line that supports the growth of a defective early region 4 deletion mutant of human adenovirus type 2. *Proc. Natl. Acad. Sci. USA* **80**: 5838-5386.
Weinberg, R.A. 1991. Tumor suppressor genes. *Science* **254**: 1138-1146.
Yates, J.L., N. Warren, and B. Sugden. 1985. Stable replication of plasmids derived from Epstein-Barr virus in various mammalian cells. *Nature* **313**: 812-815.
Zhang, W.-W., X. Fang, C.D. Branch, W. Mazur, B.A. French, and J.A. Roth. 1994. Generation and identification of recombinant adenovirus by liposome-mediated transfection and PCR analysis. *BioTechniques* **15**: 868-872.
Zubrzycka-Gaarn, E.E., D.E. Bulman, G. Karpati, A.H.M. Burghes, B. Belfall, H.J. Klamut, J. Talbot, R.S. Hodges, P.N. Ray, and R.G. Worton. 1988. The Duchenne muscular dystrophy gene product is localized in sarcolemma of human skeletal muscle. *Nature* **333**: 466-469.

## Claims

1. A virion particle containing a packaged adenovirus vector construct, wherein said vector construct comprises an adenoviral inverted terminal repeat and an adenoviral packaging signal but lacks at least a portion of the coding regions for
(i) the adenoviral products E1A, E1B, and E3; and
(ii) at least one of the adenoviral products selected from the group consisting of E2, E4, L1, L2, L3, L4 and L5,
and wherein said virion particle is modified to incorporate a cell targeting agent that is encoded by a gene inserted into the vector construct.

2. The virion particle of claim 1, wherein said vector construct lacks the coding regions for adenoviral products E1A, E1B, E2 and E3.

3. The virion particle of claim 1, wherein said vector construct lacks the coding regions for adenoviral products E1A, E1B, E3 and E4.

4. The virion particle of claim 1, wherein said vector construct lacks the coding regions for adenoviral products E1A, E1B, E3, E4 and L1-5.

5. The virion particle of claim 1, wherein said vector construct lacks the coding regions for adenoviral products E1A, E1B, E2, E3, and E4.

6. The virion particle of claim 1, wherein said vector construct lacks the coding regions for adenoviral products E1A, E1B, E2, E3 and L1-5.

7. The virion particle of claim 1, wherein said vector construct lacks the coding regions for adenoviral products E1A, E1B, E2, E3, E4, L1, L2, L3, L4 and L5.

8. The virion particle of claim 7, wherein said vector construct lacks all adenoviral coding regions.

9. A virion particle containing a packaged adenovirus vector construct, wherein said vector construct comprises an adenoviral inverted terminal repeat and an adenoviral packaging signal and a non-functional, non-immunogenic form of
(i) the adenoviral products E1A, E1B and E3; and
(ii) at least one of the adenoviral products selected from the group consisting of E2, E4, L1, L2, L3, L4 and L5,
and wherein said virion particle is modified to incorporate a cell targeting agent that is encoded by a gene inserted into the vector construct.

10. The virion particle of claim 1, wherein said vector construct further comprises a heterologous DNA of at least 10 kB.

11. The virion particle of claim 1, wherein said vector construct further comprises a heterologous DNA of at least 15 kB.

12. The virion particle of claim 11, wherein said vector construct further comprises a heterologous DNA of at least 20 kB.

13. The virion particle of claim 12, wherein said vector construct further comprises a heterologous DNA of at least 30 kB.

14. The virion particle of claim 13, wherein said vector construct further comprises a heterologous DNA of at least 35 kB.

15. The virion particle of claim 10, wherein said vector construct further comprises a promoter and wherein said heterologous DNA is operably linked to said promoter.

16. The virion particle of any of the claims 10 to 15, wherein said heterologous DNA encodes a tumor supressor.

17. The virion particle of any of the claims 10 to 15, wherein said heterologous DNA encodes a product involved with cystic fibrosis.

18. The virion particle of any of the claims 10 to 15, wherein said heterologous DNA encodes a product involved with Duchenne muscular dystrophy.

19. The virion particle of any of the claims 10 to 15, wherein said heterologous DNA encodes an antisense construct.

20. The virion particle of claim 15, wherein said promoter is an adenoviral major late promoter.

21. The virion particle of claim 15, wherein said promoter is a heterologous, cell-specific promoter.

22. The virion particle of any of the claims 1 to 21, wherein the gene encoding the cell targeting agent is inserted into a viral capsid gene.

23. The virion particle of any of the claims 1 to 22, wherein the cell targeting agent is an antibody.

24. The virion particle of any of the claims 1 to 22, wherein the cell targeting agent is a cell receptor recognition peptide.

25. The virion particle of any of the claims 1 to 22, wherein the cell targeting agent is a binding site peptide.

26. The virion particle of any of the claims 1 to 22, wherein the cell targeting agent is a ligand peptide.

27. The virion particle of any of the claims 1 to 22, wherein the cell targeting agent gene encodes an Fc binding region.

28. The virion particle of any of the claims 1 to 22, wherein the cell targeting agent gene encodes a ligand binding site.

29. The virion particle of any of the claims 1 to 22, wherein the cell targeting agent gene encodes a cell specific epitope.

## Patentansprüche

1. Virionpartikel, enthaltend ein verpacktes Adenovirusvektorkonstrukt, wobei das Vektorkonstrukt eine adenovirale invertierte terminale Sequenzwiederholung und ein adenovirales Verpackungssignal umfasst, aber wenigstens einen Teil der kodierenden Regionen für
(i) die adenoviralen Produkte E1A, E1B und E3; und
(ii) wenigstens eines der adenoviralen Produkte, ausgewählt aus der Gruppe bestehend aus E2, E4, L1, L2, L3, L4 und L5,
nicht aufweist, und wobei das Virionpartikel so modifiziert ist, dass es ein Agens zur gezielten Ansteuerung einer Zelle (cell targeting agent) einschließt, welches von einem Gen kodiert wird, das in das Vektorkonstrukt insertiert ist.

2. Virionpartikel nach Anspruch 1, wobei das Vektorkonstrukt die kodierenden Regionen für die adenoviralen Produkte E1A, E1B, E2 und E3 nicht aufweist.

3. Virionpartikel nach Anspruch 1, wobei das Vektorkonstrukt die kodierenden Regionen für die adenoviralen Produkte E1A, E1B, E3 und E4 nicht aufweist.

4. Virionpartikel nach Anspruch 1, wobei das Vektorkonstrukt die kodierenden Regionen für die adenoviralen Produkte E1A, E1B, E3, E4 und L1-5 nicht aufweist.

5. Virionpartikel nach Anspruch 1, wobei das Vektorkonstrukt die kodierenden Regionen für die adenoviralen Produkte E1A, E1B, E2, E3 und E4 nicht aufweist.

6. Virionpartikel nach Anspruch 1, wobei das Vektorkonstrukt die kodierenden Regionen für die adenoviralen Produkte E1A, E1B, E2, E3 und L1-5 nicht aufweist.

7. Virionpartikel nach Anspruch 1, wobei das Vektorkonstrukt die kodierenden Regionen für die adenoviralen Produkte E1A, E1B, E2, E3, E4, L1, L2, L3, L4 und L5 nicht aufweist.

8. Virionpartikel nach Anspruch 7, wobei das Vektorkonstrukt alle adenoviralen kodierenden Regionen nicht aufweist.

9. Virionpartikel, enthaltend ein verpacktes Adenovirusvektorkonstrukt, wobei das Vektorkonstrukt eine adenovirale invertierte terminale Sequenzwiederholung und ein adenovirales Verpackungssignal und eine nichtfunktionelle, nichtimmunogene Form von
(i) den adenoviralen Produkten E1A, E1B und E3; und
(ii) wenigstens einem der adenoviralen Produkte, ausgewählt aus der Gruppe bestehend aus E2, E4, L1, L2, L3, L4 und L5,
umfasst, und wobei das Virionpartikel so modifiziert ist, dass es ein Agens zur gezielten Ansteuerung einer Zelle einschließt, welches von einem Gen kodiert wird, das in das Vektorkonstrukt insertiert ist.

10. Virionpartikel nach Anspruch 1, wobei das Vektorkonstrukt außerdem eine heterotoge DNA von wenigstens 10 kB umfasst.

11. Virionpartikel nach Anspruch 1, wobei das Vektorkonstrukt außerdem eine heterologe DNA von wenigstens 15 kB umfasst.

12. Virionpartikel nach Anspruch 11, wobei das Vektorkonstrukt außerdem eine heterologe DNA von wenigstens 20 kB umfasst.

13. Virionpartikel nach Anspruch 12, wobei das Vektorkonstrukt außerdem eine heterologe DNA von wenigstens 30 kB umfasst.

14. Virionpartikel nach Anspruch 13, wobei das Vektorkonstrukt außerdem eine heterologe DNA von wenigstens 35 kB umfasst.

15. Virionpartikel nach Anspruch 10, wobei das Vektorkonstrukt außerdem einen Promotor umfasst und wobei die heterologe DNA mit dem Promotor funktionell verbunden ist.

16. Virionpartikel nach einem der Ansprüche 10 bis 15, wobei die heterologe DNA für einen Tumorsuppressor kodiert.

17. Virionpartikel nach einem der Ansprüche 10 bis 15, wobei die heterologe DNA für ein Produkt kodiert, das bei der zystischen Fibrose eine Rolle spielt.

18. Virionpartikel nach einem der Ansprüche 10 bis 15, wobei die heterologe DNA für ein Produkt kodiert, das bei der Duchenne-Muskeldystrophie eine Rolle spielt.

19. Virionpartikel nach einem der Ansprüche 10 bis 15, wobei die heterologe DNA für ein Antisensekonstrukt kodiert.

20. Virionpartikel nach Anspruch 15, wobei der Promotor ein hauptsächlicher später adenoviraler Promotor ist.

21. Virionpartikel nach Anspruch 15, wobei der Promotor ein heterologer zellspezifischer Promotor ist.

22. Virionpartikel nach einem der Ansprüche 1 bis 21, wobei das Gen, das für das Agens zur gezielten Ansteuerung einer Zelle kodiert, in ein virales Kapsidgen insertiert ist.

23. Virionpartikel nach einem der Ansprüche 1 bis 22, wobei das Agens zur gezielten Ansteuerung einer Zelle ein Antikörper ist.

24. Virionpartikel nach einem der Ansprüche 1 bis 22, wobei das Agens zur gezielten Ansteuerung einer Zelle ein Zellrezeptorerkennungspeptid ist.

25. Virionpartikel nach einem der Ansprüche 1 bis 22, wobei das Agens zur gezielten Ansteuerung einer Zelle ein Bindungsstellenpeptid ist.

26. Virionpartikel nach einem der Ansprüche 1 bis 22, wobei das Agens zur gezielten Ansteuerung einer Zelle ein Ligandenpeptid ist.

27. Virionpartikel nach einem der Ansprüche 1 bis 22, wobei das Gen für das Agens zur gezielten Ansteuerung einer Zelle eine Fc-Bindungsregion kodiert.

28. Virionpartikel nach einem der Ansprüche 1 bis 22, wobei das Gen für das Agens zur gezielten Ansteuerung einer Zelle eine Ligandenbindungsstelle kodiert.

29. Virionpartikel nach einem der Ansprüche 1 bis 22, wobei das Gen für das Agens zur gezielten Ansteuerung einer Zelle ein zellspezifisches Epitop kodiert.

## Revendications

1. Virion contenant un vecteur adénoviral encapsidé recombinant, dans lequel ledit vecteur recombinant contient une répétition inverse terminale adénovirale et un signal d'encapsidation adénovirale, mais à qui il manque au moins une portion des régions codant pour
(i) les produits adénoviraux E1A, E1B et E3 ; et
(ii) au moins l'un des produits adénoviraux choisis au sein du groupe constitué de E2, E4, L1, L2, L3, L4 et L5,
et dans lequel ledit virion est modifié pour incorporer un agent de ciblage cellulaire codé par un gène inséré dans le vecteur recombinant.

2. Virion selon la revendication 1, dans lequel il manque audit vecteur recombinant les régions codant pour les produits adénoviraux E1A, E1B, E2 et E3.

3. Virion selon la revendication 1, dans lequel il manque audit vecteur recombinant les régions codant pour les produits adénoviraux E1A, E1B, E3 et E4.

4. Virion selon la revendication 1, dans lequel il manque audit vecteur recombinant les régions codant pour les produits adénoviraux E1A, E1B, E3, E4 et L1-5.

5. Virion selon la revendication 1, dans lequel il manque audit vecteur recombinant les régions codant pour les produits adénoviraux E1A, E1B, E2, E3 et E4.

6. Virion selon la revendication 1, dans lequel il manque audit vecteur recombinant les régions codant pour les produits adénoviraux E1A, E1B, E2, E3 et L1-5.

7. Virion selon la revendication 1, dans lequel il manque audit vecteur recombinant les régions codant pour les produits adénoviraux E1A, E1B, E2, E3, E4, L1, L2, L3, L4 et L5.

8. Virion selon la revendication 7, dans lequel il manque audit vecteur recombinant toutes les régions de codage adénoviral.

9. Virion contenant un vecteur adénoviral encapsidé recombinant, dans lequel ledit vecteur recombinant comprend une répétition inverse terminale adénovirale et un signal d'encapsidation adénovirale, ainsi qu'une forme non-fonctionnelle, non-immunogène
(i) des produits adénoviraux E1A, E1B et E3 ; et
(ii) d'au moins un des produits adénoviraux choisis dans le groupe constitué de E2, E4, L1, L2, L3, L4 et L5,
et dans lequel ledit virion est modifié pour incorporer un agent de ciblage cellulaire codé par un gène inséré dans le vecteur recombinant.

10. Virion selon la revendication 1, dans lequel ledit vecteur recombinant comprend en outre un ADN hétérologue d'au moins 10 kB.

11. Virion selon la revendication 1, dans lequel ledit vecteur recombinant comprend en outre un ADN hétérologue d'au moins 15 kB.

12. Virion selon la revendication 11, dans lequel ledit vecteur recombinant comprend en outre un ADN hétérologue d'au moins 20 kB.

13. Virion selon la revendication 12, dans lequel ledit vecteur recombinant comprend en outre un ADN hétérologue d'au moins 30 kB.

14. Virion selon la revendication 13, dans lequel ledit vecteur recombinant comprend en outre un ADN hétérologue d'au moins 35 kB.

15. Virion selon la revendication 10, dans lequel ledit vecteur recombinant comprend en outre un promoteur et dans lequel ledit ADN hétérologue est lié de façon fonctionnelle audit promoteur.

16. Virion selon l'une quelconque des revendications 10 à 15, dans lequel ledit ADN hétérologue code pour un suppresseur de tumeur.

17. Virion selon l'une quelconque des revendications 10 à 15, dans lequel ledit ADN hétérologue code pour un produit impliqué dans la mucoviscidose.

18. Virion selon l'une quelconque des revendications 10 à 15, dans lequel ledit ADN hétérologue code pour un produit impliqué dans la myopathie de Duchenne.

19. Virion selon l'une quelconque des revendications 10 à 15, dans lequel ledit ADN hétérologue code pour un antisens recombinant.

20. Virion selon la revendication 15, dans lequel ledit promoteur est un promoteur majeur tardif de l'adénovirus.

21. Virion selon la revendication 15, dans lequel ledit promoteur est un promoteur hétérologue spécifique des cellules.

22. Virion selon l'une quelconque des revendications 1 à 21, dans lequel le gène codant pour l'agent de ciblage cellulaire est inséré dans un gène de la capside virale.

23. Virion selon l'une quelconque des revendications 1 à 22, dans lequel l'agent de ciblage cellulaire est un anticorps.

24. Virion selon l'une quelconque des revendications 1 à 22, dans lequel l'agent de ciblage cellulaire est un peptide de reconnaissance des récepteurs cellulaires.

25. Virion selon l'une quelconque des revendications 1 à 22, dans lequel l'agent de ciblage cellulaire est un peptide de site de liaison.

26. Virion selon l'une quelconque des revendications 1 à 22, dans lequel l'agent de ciblage cellulaire est un peptide ligand.

27. Virion selon l'une quelconque des revendications 1 à 22, dans lequel le gène de l'agent de ciblage cellulaire code pour une région de liaison Fc.

28. Virion selon l'une quelconque des revendications 1 à 22, dans lequel le gène de l'agent de ciblage cellulaire code pour un site de liaison du ligand.

29. Virion selon l'une quelconque des revendications 1 à 22, dans lequel le gène de l'agent de ciblage cellulaire code pour un déterminant antigénique spécifique des cellules.
